# EUROPEAN PATENT APPLICATION

(11) **EP 4 589 322 A1**
(43) Date of publication of application: **23.07.2025**
(21) Application number: 24152392.7
(22) Date of filing: 17.01.2024
(51) Int. Cl.: G01R 33/483, G01R 33/50, G01R 33/561

(54) **COMPUTER-IMPLEMENTED METHOD FOR DETERMINING A T1RHO MAP, MAGNETIC RESONANCE DEVICE, COMPUTER PROGRAM AND ELECTRONICALLY READABLE STORAGE MEDIUM**

(71) Applicant: Siemens Healthineers AG, 91301 Forchheim (DE); King's College London, London WC2R 2LS (GB)
(72) Inventor: Roujol, Sebastien, Kingston upon Thames, KT2 6JB (GB); Tyler, Andrew, London, SW20 0UF (GB); Kunze, Karl-Philipp, London, SW10 9JR (GB)
(74) Representative: Siemens Healthineers Patent Attorneys

(57) **Abstract**

Computer-implemented method for determining a T1ρ map, using a magnetic resonance device (9), wherein magnetic resonance data are acquired from multiple slices of an imaging volume of a patient for different spin lock times provided by a T1ρ preparation module (1), which comprises at least one spin lock pulse (2, 3), and the T1ρ map is reconstructed from the magnetic resonance data of the different spin lock times, wherein the magnetic resonance data of each slice are acquired during a same breath hold interval (7),
characterized in that
- a slice selective T1ρ preparation module (1) is used as the slice selective T1ρ preparation module (1), and
- magnetic resonance data of at least two slices are acquired in at least one of the breath hold intervals (7).

## Description

The invention concerns a computer-implemented method for determining a T1ρ map using a magnetic resonance device, wherein magnetic resonance data are acquired from multiple slices of an imaging volume, in particular comprising the heart, of a patient for different spin lock times provided by a T1ρ preparation module, which comprises at least one spin lock pulse, and the T1ρ map is reconstructed from the magnetic resonance data of the different spin lock times, wherein the magnetic resonance data of each slice are acquired during a same breath hold interval. The invention further concerns a magnetic resonance device, a computer program and an electronically readable storage medium.

Established cardiovascular magnetic resonance (CMR) uses late gadolinium enhanced cardiac magnetic resonance imaging (LGE-CMR) as the gold standard technique for the assessment of cardiac scar formation following myocardial infarction, see, for example, Kim et al., "Relationship of MRI delayed contrast enhancement to irreversible injury, infarct age, and contractile function", Circulation 100(1999), pages 1992-2002. However, such techniques using a contrast agent have a number of drawbacks, in particular the incompatibility of the contrast agent injection with patients with kidney disease, and the increased clinical workload of adding the injection to the scan. Hence, native imaging methods have been developed. For example, native T1 contrast techniques, known by the names of "MyoMaps" for Siemens AG, München, Germany, and "CVWorks" for General Electrics, Boston, USA, have been proposed for assessing scarred tissue.

In recent works, however, it has been shown that native T1ρ contrast offers intrinsically superior information, since it is directly sensitive to the interaction of macromolecules formed in fibrosis and water, as opposed to T1, see the review article by A. Bustin et al., "Magnetic resonance myocardial T1ρ mapping", JCMR 25(2023), article number 34. While T1 and T2 describe spin-lattice and spin-spin relaxations, respectively, T1ρ relaxation time describes spin-lattice relaxation in the rotation frame at the presence of an external radio frequency (rf) pulse in the transverse plane. In other words, it is the time constant for the decay along the radio frequency field (B1). To measure T1ρ, at least one spin lock pulse is applied in a T1ρ preparation module for different spin locking times.

Myocardial T1ρ mapping has been validated for myocardial scar assessment, see, for example van Oorschot et al, "Endogenous assessment of chronic myocardial infarction with T1p-mapping in patients", JCMR 16 (2014), article number 104. However, this approach uses non-slice-selective spin lock pulses to produce T1ρ weighted images used in map formation. Furthermore, magnetization recovery times are provided between acquisitions of different spin lock times. This leads to the significant drawback that only one slice can be imaged per breath-hold. This is an important limitation for myocardial scar characterization, where full imaging coverage of the left ventricle is required. Currently, myocardial T1ρ mapping with full coverage would thus require multiple, for example 12-16, breath-hold scans, significantly prolonging the CMR examination and reducing patient comfort.

Another challenge of myocardial T1ρ mapping is the sensitivity of continuous wave T1ρ spin lock pulses to main field (B0) and RF field (B1) inhomogeneity. A different class of T1ρ spin lock pulses which rely on the T1ρ magnetization decay during an adiabatic pulse can also be applied to T1ρ mapping, as proposed by Michaeli et al., "T1ρ MRI contrast in the human brain: modulation of the longitudinal rotating frame relaxation shutter-speed during an adiabatic RF pulse", JMR 181 (2006), pages 135-147, and have recently been implemented for cardiac T1ρ mapping, as described by Coletti et al. in "Robust cardiac T1ρ mapping at 3T using adiabatic spin-lock preparations", MRM 90 (2023), pages 1363-1379. Image artefacts were significantly reduced.

In concrete known techniques, four or five T1ρ-weighted images of a single slice, in particular a cardiac slice, of a slice stack forming the imaging volume are acquired during each breath hold interval, for example 10 to 15 seconds, roughly equating to 10 to 16 heart cycles (heart beats). The relaxation time T1ρ is determined by fitting these magnetic resonance data to a known T1ρ decay constant equation (model). Each T1ρ weighted image acquisition is separated by two or three heart cycles to allow partial recovery of the longitudinal magnetization, following saturation from the imaging readout. The spin-lock pulses are typically based on a tip-down, continuous wave (CW) RF spin lock, tip-up structure, which is intrinsically non-selective. Alternatively, spin-lock can be achieved with adiabatic pulses, as has been demonstrated recently for CMR in the cited article by Coletti et al., MRM 90 (2023), pages 1363-1379. These adiabatic pulses solve one of the key issues for T1ρ mapping, namely avoiding the sensitivity of CW irradiation to field inhomogeneities.

Myocardial T1ρ mapping can alternatively be achieved using three-dimensional acquisition techniques, as discussed by Bustin et al. in the above-cited article in JCMR 25 (2023), 34. However, this approach also results in long acquisition times, even in the presence of high acceleration, see, for example, Qi et al., "Respiratory motion-compensated high resolution 3D whole-heart T1ρ mapping", JCMR 22 (2021), article number 12.

It is an object of the current invention to allow for faster acquisition, in particular high quality, in determination of T1ρ maps, in particular cardiac T1ρ maps.

This object is achieved by providing a computer-implemented method according to claim 1, a magnetic resonance device according to claim 13, a computer program according to claim 14 and an electronically readable storage medium according to claim 15. Advantageous embodiments are described by the dependent claims.

In a method as initially described, according to the invention,
- a slice selective T1ρ preparation module is used as the T1ρ preparation module and
- magnetic resonance data of at least two slices are acquired in at least one of the breath hold intervals.

A slice selective pulse has a spatial excitation profile which, in slice direction, covers a target slice as accurately as possible. In particular, there is no relevant effect on the other slices to be acquired in the same breath hold interval, such that magnetic resonance data for several spin lock times, in particular all spin lock times to be used, can be measured. Preferably, the T1ρ preparation module comprises at least one slice selection gradient pulse for the at least one spin lock pulse. In this manner, slice selectivity is established. Advantageously, as in principle known for slice selective preparation, the preparation module may also comprise a spoiler gradient pulse played out after the spin lock pulses.

It is hence proposed to acquire magnetic resonance data for at least to slices of the T1ρ map in each breath-hold. Hence, a smaller number of breath hold intervals is required, at least halving the time to acquire the full stack of slices, in particular a stack with full coverage of the heart of the patient. This is enabled by using a slice selective T1ρ preparation module (also called T1ρ contrast module). It has been shown in experiments that even better precision may also result. The improvement in speed over existing T1ρ mapping acquisitions, without reduction in precision, can significantly aid incorporation of T1ρ mapping into the clinical workflow for cardiac MRI examinations in patients with suspected myocardial scars, for example following infarction. Experiments have shown that even an increase in precision can be achieved.

After the magnetization in the slice has been prepared by the T1ρ preparation module, as known, a slice selective imaging sequence, also comprising at least one RF excitation pulse, is used to acquire the magnetic resonance data. This imaging sequence may also be called readout or measurement module. Hence, in other words, a measurement module is used after the preparation module to acquire the magnetic resonance data. The measurement module may preferably be or comprise a bSSFP (balanced steady-state free precession) readout sequence. However, in principle, other kinds of imaging sequences may also be used, comprising Gradient Echo (GE), Spin Echo (SE), or Echo Planar Imaging (EPI) sequences. Any desired k-space trajectory may be employed, comprising preferred cartesian trajectories as well as radial or spiral trajectories.

Regarding reconstruction of the T1ρ map, in particular determining T1ρ images for the slices, known approaches may be used. For example, T1ρ values may be determined by parametric mapping based in two- or three-parameter models describing T1ρ as exponential decay constant for the magnetic resonance signal, that is, for example, S(t) being proportional to exp(-t/T1ρ). In preferred embodiments, dictionary-based estimation of T1ρ values is employed during reconstruction of the T1ρ map, in particular using Bloch simulations and/or two- or three-parameter models. Dictionary entries may be pre-calculated from the Bloch simulation and/or the respective model for the spin lock times also used during measurement. The measured magnetic resonance data for the different spin lock times and each voxel, that is, the fingerprint of the voxel, are then matched against the pre-calculated fingerprints of the dictionary, providing an estimate of T1ρ. Dictionary matching is a preferred approach, since additional, in particular complex, interactions and/or effects can be taken into account.

In preferred embodiments, each slice selective preparation module may comprise at least one pair of tip-up and tip-down spin lock pulses, in particular 180° pulses, wherein the number of pairs defines the respective spin lock time. That is, the T1ρ preparation module comprises n pairs of tip-up and tip-down spin lock pulses, wherein n determines the spin lock time prepared. In embodiments, the tip-up and the tip-down pulse may have opposing amplitudes. For example, each pair of spin lock pulses may provide a spin lock time duration of 10 to 100 ms, for example 20 or 20.5 ms. Here, advantageously, each spin lock pulse may be a slice specific adiabatic inversion pulse, in particular a hyperbolic secant pulse. Such a pulse design has generally already been proposed in the cited article by Coletti et al., and proven to be advantageous in T1ρ mapping, since sensitivity to B0 and B1 inhomogeneities is reduced. These spin lock pulses can be applied slice selectively by using a slice selection gradient pulse. Hence, preferably, the preparation module may for example be based on a series of slice-selective 180° hyperbolic secant (HS) tip down/up spin lock pulse pairs. For such spin lock pulses, outstanding results have been achieved in experiments.

The concrete parameters of the pulses may be optimized for T1ρ measurement as principally known in the art, see, for example, the article by Coletti et al.. For example, the hyperbolic secant pulses may have a β constant describing the width of the pulse bell of 0 to 10, in particular 5 to 7. β, which may be understood as a truncation factor for the pulse shape, may have a value of 0 to 10, although higher values may still be usable. Preferably, a maximum B1 field frequency of 400 to 1000 Hz, in particular 700 to 900 Hz, and/or a bandwidth of 1000 to 2000 Hz, in particular 1400 to 1600 Hz, may be used. B1Max, describing the maximum B1 field of the spin lock pulse, depends on the RF capabilities of the magnetic resonance device and SAR limitations. Typically, 400-1000Hz may be used for cardiac T1ρ mapping, with lower values producing little scar contrast. The bandwidth contributes to the effective spin lock frequency.

Concrete examples of HS pulses, to which slice selectivity is applicable, further comprise HS-1 or HS-4 pulses. It is, however, noted that, in principle, any type of spin lock pulse which can be applied slice selectively, can be used in the current invention. For example, GOIA-W pulses can be used as spin lock pulses in the T1ρ preparation module of the invention.

In concrete embodiments, magnetic resonance data for each slice are measured at least for 0, 2 and 4 pairs of spin lock pulses. Using the spin lock duration example of 20.5 ms mentioned above, spin lock times of 0 ms, 41 ms, and 82 ms can be achieved, covering a relevant range for high quality fitting to a model or dictionary matching.

As already mentioned, the current invention is particularly advantageous regarding measurements of the human heart, in particular regarding myocardium scar assessment. Hence, preferably, the imaging region is a cardiac region of the patient, in particular comprising the heart. Such a region is typically subject to two sources of motion, namely heart motion and breathing motion.

Regarding breathing motion, the acquisition time comprises multiple breath hold intervals, which may have a defined, not too long breath hold duration to not overexert the patient. For example, breath hold durations of 10 to 15 seconds may be used. Since a heart cycle comprising one heart beat can be said to take about one second, 10 to 15 heart beats may be covered in one breath hold interval. In particular, the breath hold duration may be adapted to a current heart rate of the patient to provide a certain number of heart cycles in each breath hold interval.

To deal with heart motion, preferably, each acquisition block for a specific slice and spin lock time may be triggered at a predetermined position in the heart cycle of the patient. For example, triggering may be in the end-diastolic phase of the heart cycle. In this manner, due to the breath-hold, the breathing motion state for all slices acquired during a breath hold interval is the same, and the magnetic resonance data all relate to at least similar heart motion states. The breath hold interval can hence be understood as being divided into heart cycle sub-intervals, wherein, in each heart cycle sub-interval, magnetic resonance data for a specific slice and spin lock time can be acquired. To acquire magnetic resonance data for multiple slices, their acquisition blocks are distributed to these heart cycle sub-intervals.

In principle, it is conceivable to acquire magnetic resonance data of different spin lock times for the same slice in, in particular directly, adjacent heart cycles, allowing only very short recovery times for the magnetization. However, in such a case, the magnetization has to be tracked over the respective heart cycles, for example by using the Block equations. If, for example, in the first acquisition block, the starting relative magnetization is 1, the magnetization may only recover to 0.8 at the beginning of the next acquisition block in the next heart cycle, which has to be accounted for when evaluating the magnetic resonance data. However, tracking the magnetization like this is cumbersome and prone to errors, so that it is preferred to provide a sufficient recovery time for the magnetization, in particular at least essentially three or four heart cycles, to return to at least essentially its full value.

Hence, in especially preferred embodiments, the acquisition blocks for the different slices of a breath hold interval may be interleaved, such that, for at least the number of heart cycles in which magnetic resonance data for other slices are acquired, a recovery time (of more than one heart cycle) between acquisitions for different spin lock times is provided. Hence, the acquisition of magnetic resonance data for at least two slices while providing sufficient recovery time is achieved by interleaving acquisition of at least two slices during the breath-hold, which is made possible by the use of the slice selective T1ρ preparation modules.

In embodiments, additional heart cycles, in which no acquisition occurs, may be used to prolong the recovery times. In other words, two acquisition blocks for different slices may be spaced by at least one heart cycle sub interval, which is not used for an acquisition block. In a concrete, advantageous example, magnetic resonance data for two slices may be acquired in one breath hold interval, wherein each of the interleaved acquisition blocks are separated by one heart cycle, in which no acquisition occurs, such that a recovery time of about four heart cycles, comprising three additional heart cycles, results for each slice. Acquisition time for the whole imaging region may be halved.

However, to provide further acceleration of the acquisition and/or acquire magnetic resonance data for more spin lock times, the acquisition blocks for all slices may also be performed in immediately adjacent heart cycles, such that the recovery time comprises a number of heart cycles equal to the number of slices acquired in one breath hold interval. For example, in a breath hold interval covering twelve heart cycles and four slices A, B, C, D, the acquisition order [A, B, C, D, A, B, C, D, A, B, C, D] can be used to acquire magnetic resonance data for three spin lock times in one breath hold interval. This accounts for four times acceleration of the acquisition, while still providing a recovery time of four heart cycles. It is, however, noted that a narrower excitation profile in slice direction may be required as in the case of two slices per breath-hold to prevent slice crosstalk.

Preferably, as already indicated, a correction regarding effects of insufficient magnetization recovery between acquisition blocks for different spin lock times is performed based on at least one Bloch simulation and/or on at least one experimental result, in particular of experiments to determine empirical values used for correction. Even if longer recovery times are provided, full recovery may not be achieved, such that a remaining effect may result in artifacts and/or generally lower quality of the B1ρ map. Hence, expected deviations from the full equilibrium magnetization may be calculated using Bloch simulation and used to correct the magnetic resonance data of later acquisition blocks.

Despite triggering and breath-hold, there may be differences in the heart motion state and/or breathing motion state, respectively. Hence, preferably, motion correction may be applied during reconstruction, in particular for heart motion. For example, correction approaches based on non-rigid registration of slices may be used. Other options may include movement sensors, MR navigator scans, and the like.

It is noted that the slices to be acquired in the same breath hold interval are preferably chosen such that the distance between the slices in the slice selection direction is as large as possible, to minimize the effect of the preparation modules (and other slice selective pulses of the acquisition blocks) onto the other slices. For example, for two slices to be acquired in the same breath hold interval and an even total number of slices in the slice stack, the distance of each pair of slices to be acquired in the same breath hold interval may be half the total number.

The invention further concerns a magnetic resonance device, comprising a control device configured to perform a method according to the invention. All features and remarks regarding the method according to the invention can be applied to the magnetic resonance device according to the invention and vice versa.

The magnetic resonance device may comprise a main magnet unit having a bore for receiving the patient. A main magnet of the main magnet unit generates the main magnetic field (B0 field). A gradient coil arrangement is provided to output gradient pulses, and a radio frequency coil arrangement may be controlled to generate radio frequency pulses.

The control device may comprise at least one processor and at least one storage means. Functional units may be implemented in hardware and/or software and be configured to perform steps of the method according to the invention. In particular, the control device may comprise, as in principle known, a sequence unit for controlling the gradient coil arrangement and the radio frequency coil arrangement to generate pulses according to a magnetic resonance sequence and acquire respective magnetic resonance data, and a reconstruction unit to reconstruct image data sets, in particular also comprising maps, from acquired magnetic resonance data. In the magnetic resonance device according to the invention, the sequence unit is configured to acquire magnetic resonance data from multiple slices of an imaging volume of a patient for different spin lock times provided by a slice selective T1ρ preparation module, which comprises at least one slice selective spin lock pulse, wherein magnetic resonance data of at least two slices are acquired in a same breath hold interval. The reconstruction unit is configured to reconstruct a T1ρ map from the magnetic resonance data of the different spin lock times.

A computer program according to the invention can be directly loaded into a storage means of a control device of a magnetic resonance device and comprises program means such that, when the computer program is executed in the control device, the control device is caused to perform the steps of a method according to the invention. The computer program may be stored on an electronically readable storage medium, which thus comprises control information, which comprises a computer program according to the invention, stored thereon such that, when the storage medium is used in a control device of a magnetic resonance device, the control device is configured to perform a method according to the invention. The storage medium may be a non-transient storage medium, for example a CD ROM.

Other objects and features of the present invention will become apparent from the following detailed description considered in conjunction with the accompanying drawings. The drawings, however, are only principle sketches designed solely for the purpose of illustration and do not limit the invention. The drawings show:
- Fig. 1: a pulse sequence diagram showing a T1ρ preparation module and the associated measurement module,
- Fig. 2: a first acquisition scheme,
- Fig. 3: a second acquisition scheme,
- Fig. 4: an embodiment of a magnetic resonance device according to the invention, and
- Fig. 5: the functional structure of a control device of the magnetic resonance device.

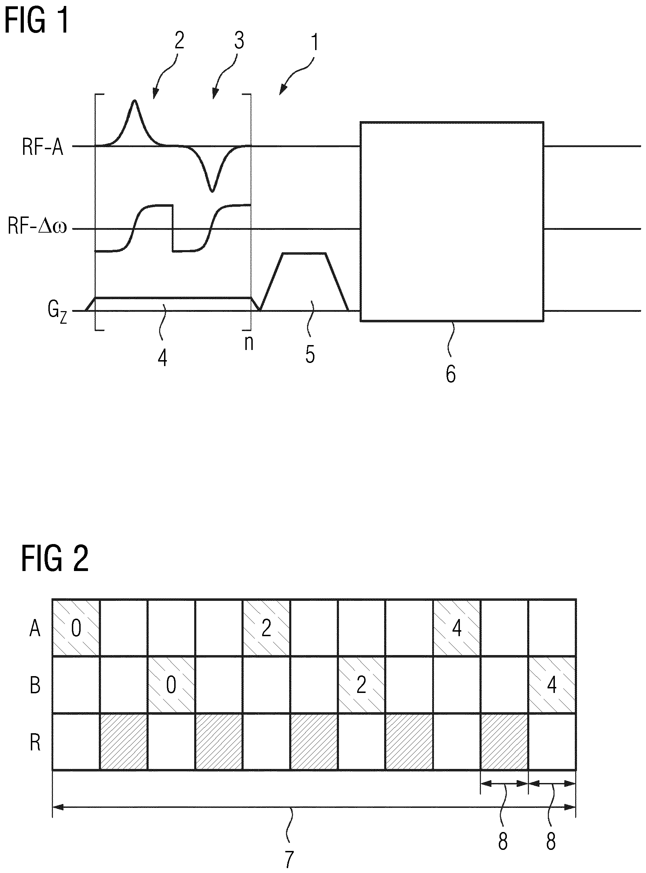

Fig. 1 shows an embodiment of a slice selective T1ρ preparation module 1 as it can be used in a method according to the invention. The T1ρ preparation module 1 comprises a sequence of n pairs of spin lock pulses 2, 3, as indicated by the bracket labelled "n". The upper graph of Fig. 1 shows the radio frequency amplitude (RF-A), the middle graph the RF frequency shift (RF Δω). As can be seen, both spin lock pulses 2, 3 are adiabatic pulses, in this case 180° hyperbolic secant pulses (HS pulses). The spin lock pulse 2 is a tip down pulse, the spin lock pulse 3 a tip up pulse, forming a tip down-tip up pair. In a concrete example, the bandwidth is 1.5 kHz, β = 6, and the maximum B1 field parameter is Bimax = 825 Hz. Due to a slice selection gradient pulse 4 shown in the lower graph, the pulses 2, 3 are slice selective. The T1ρ preparation module also comprises a spoiler pulse 5. In this case, the slice selection direction is z.

After the magnetization has been prepared by the T1ρ preparation module 1, it is measured using a measurement module 6, in this case comprising a bSSFP imaging sequence.

The number n of pairs used defines the respective spin lock time applied for preparation. In the following concrete embodiments, concerning T1ρ mapping of the heart of a patient in an imaging volume comprising a stack of slices, multiple breath hold intervals are used, each covering a defined number of heart beats and, thus, heart cycles. The slice selectivity of the T1ρ preparation module 1 allows acquisition of all magnetic resonance data of different spin lock times for multiple slices in one single breath hold interval. Magnetic resonance data for a spin lock time is acquired in an acquisition block, which, for a spin lock time of 0, does not comprise a T1ρ preparation module 1. In any other case, the acquisition block comprises a T1ρ preparation module 1 and a measurement module 6. Further pulses and/or modules may be included, for example fat saturation pulses and/or modules. In the exemplary embodiments described here, the number n of pairs used is 0, 2, and 4, generating spin lock times of 0, 41, and 82 ms. Each acquisition block is ECG triggered at the end-diastolic phase.

In the first example shown in Fig. 2, the magnetic resonance data for two slices A and B are acquired in one breath hold interval 7 of eleven seconds, which comprises eleven heart cycles 8. The upper row shows acquisition blocks for slice A, the middle row acquisition blocks for slice B. The numbers in the respective boxes indicate acquisition blocks of the respective slice A, B and the number of pairs and thus spin lock time. Acquisition of the two slices is interleaved to ensure sufficient recovery gap between T1ρ preparation modules 1 of the same slice A, B. The lowest set of boxes labelled R refers to heart cycles without any acquisition block, which are used to prolong the recovery gap between acquisition blocks of the same slice A, B. In this case, the recovery time is essentially four heart cycles.

After the magnetic resonance data for all slices have been acquired, motion correction is applied to the magnetic resonance data, that is, the T1ρ weighted images acquired using the measurement module 6. For example, non-rigid registration between slices can be performed.

T1ρ maps are then reconstructed from the three T1ρ weighted images of the different spin lock times using a two parameter model dictionary based approach. To create the dictionary, a Bloch simulation estimate of the cardiac magnetic resonance signal with infinite spin-lock time is determined to calculate T1ρ for different fingerprints. In the reconstruction process, Bloch simulations may also optionally be used to correct for insufficient recovery.

The performance of this first embodiment was evaluated against a reference technique in six healthy volunteers at 1.5 T. The reference technique had the same bSSFP measurement module and a T1ρ preparation module based on non-selective adiabatic inversion pulses (i.e. without slice selection gradient 4), acquiring three T1ρ weighted images of a single slice in one breath-hold interval 7 for a reference T1ρ map.

Subject-wise analysis showed that the first embodiment had a significantly lower T1ρ (123±8 ms vs. 13217 ms, p=0.02), significantly better precision (17.0±2.7 ms vs. 22.6±4.0 ms, p = 0.01), and not significantly different intra-subject myocardial segment variability (8.2±3.9% vs 8.213.9%, p = 0.94) with respect to the reference technique.

The first embodiment provides a two-fold acceleration for multislice myocardial T1ρ mapping, with significantly better subject-wise precision and no significant difference in myocardial segment variability, compared to the reference sequence.

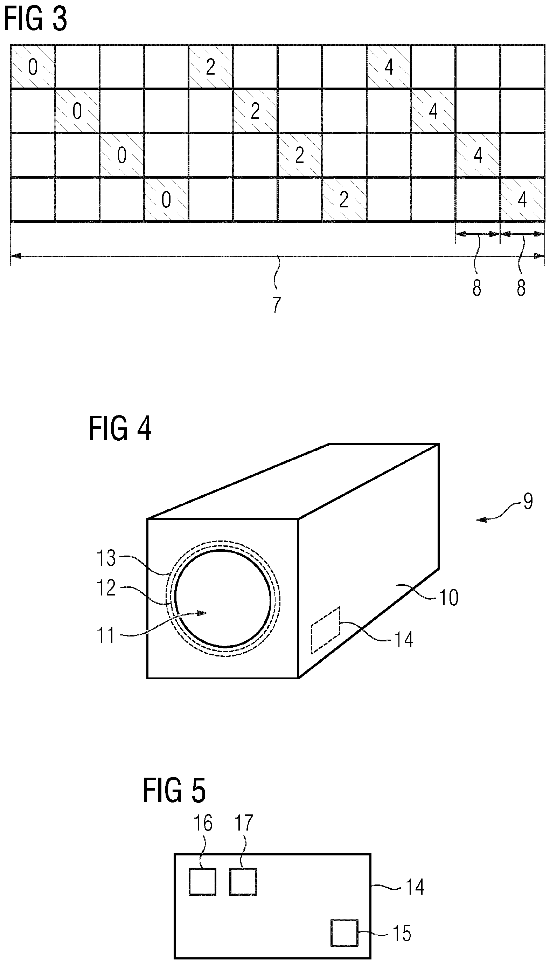

However, given a sufficiently narrow excitation profile in slice selection direction of the T1ρ preparation module, even more slices can be acquired in the same breath hold interval 7. For example, Fig. 3 illustrates a second embodiment, wherein four slices A, B, C, D are acquired in a breath hold interval 7 covering twelve heart cycles 8. Here, acquisition blocks for all slices A, B, C, D are performed in immediately adjacent heart cycles. However, due to the four slices A, B, C, D, the recovery time is still essentially four heart cycles.

Reconstruction can be performed as in the first embodiment.

Fig. 4 shows a magnetic resonance device 9 according to the invention. The magnetic resonance device 9 comprises a main magnet unit 10 having a cylindrical bore 11, into which a patient can be transported using a patient table (not shown). The main magnet unit 10 comprises an, in particular superconducting, main magnet. Encircling the bore, the magnetic resonance device 9 further comprises a gradient coil arrangement 12 and a radio frequency coil arrangement 13. Furthermore, local coils may be used with the magnetic resonance device 9.

The operation of the magnetic resonance device 9 is controlled by a control device 14, which is configured to perform a method according to the invention.

As can be seen from Fig. 5, the control device 14 comprises a storage means 15, a sequence unit 16 and a reconstruction unit 17. The sequence unit is adapted to acquire magnetic resonance data according to the acquisition schemes shown in Fig. 2 and Fig. 3, and possible further acquisition schemes as discussed herein. The magnetic resonance data acquired in this manner can be processed by the reconstruction unit, which determines a T1ρ map therefrom.

Although the present invention has been described in detail with reference to the preferred embodiment, the present invention is not limited by the disclosed examples from which the skilled person is able to derive other variations without departing from the scope of the invention.

Independent of the grammatical term usage, individuals with male, female or other gender identities are included within the term.

## Claims

1. Computer-implemented method for determining a T1ρ map, using a magnetic resonance device (9), wherein magnetic resonance data are acquired from multiple slices of an imaging volume of a patient for different spin lock times provided by a T1ρ preparation module (1), which comprises at least one spin lock pulse (2, 3), and the T1ρ map is reconstructed from the magnetic resonance data of the different spin lock times, wherein the magnetic resonance data of each slice are acquired during a same breath hold interval (7),
**characterized in that**
- a slice selective T1ρ preparation module (1) is used as the slice selective T1ρ preparation module (1), and
- magnetic resonance data of at least two slices are acquired in at least one of the breath hold intervals (7).

2. Method according to claim 1, **characterized in that** each slice selective preparation module (1) comprises at least one pair of tip-up and tip-down spin lock pulses (2, 3), wherein the number of pairs defines the respective spin lock time.

3. Method according to claim 2, **characterized in that** each spin lock pulse (2, 3) is a slice specific adiabatic inversion pulse, in particular a hyperbolic secant pulse.

4. Method according to claim 3, **characterized in that** the hyperbolic secant pulses have a β constant describing the width of the pulse bell of 0 to 10, in particular 5 to 7, and/or a maximum B1 field frequency of 400 to 1000 Hz, in particular 700 to 900 Hz, and/or a bandwidth of 1000 to 2000 Hz, in particular 1400 to 1600 Hz.

5. Method according to any of the claims 2 to 4, **characterized in that** magnetic resonance data for each slice are measured at least for 0, 2 and 4 pairs of spin lock pulses (2, 3).

6. Method according to one of the preceding claims, **characterized in that** the imaging region is a cardiac region of the patient, in particular comprising the heart.

7. Method according to one of the preceding claims, **characterized in that** each acquisition block for a specific slice and spin lock time is triggered at a predetermined position in the heart cycle (8) of the patient.

8. Method according to claim 7, **characterized in that** the acquisition blocks for the different slices of a breath hold interval (7) are interleaved, such that, for at least the number of heart cycles (8) in which magnetic resonance data for other slices are acquired, a recovery time between acquisitions for different spin lock times is provided.

9. Method according to claim 8, **characterized in that** additional heart cycles (8), in which no acquisition occurs, are used to prolong the recovery times.

10. Method according to claim 8, **characterized in that** the acquisition blocks for all slices are performed in immediately adjacent heart cycles (8), such that the recovery time comprises a number of heart cycles (8) equal to the number of slices acquired in one breath hold interval (7).

11. Method according to one of the preceding claims, **characterized in that** a correction regarding effects of insufficient magnetization recovery between acquisition blocks for different spin lock times is performed based on at least one Bloch simulation and/or at least one experimental result.

12. Method according to one of the preceding claims, **characterized in that** the T1ρ preparation module (1) comprises at least one slice selection gradient pulse (4) for the at least one spin lock pulse (2, 3).

13. Magnetic resonance device (9), comprising a control device (14) configured to perform a method according to one of the preceding claims.

14. Computer program, which, when it is executed on a control device (14) of a magnetic resonance device (9), performs a method according to any of the claims 1 to 12.

15. Electronically readable storage medium, on which a computer program according to claim 14 is stored.
